(19)

Europäisches Patentamt
European Patent Office
Office européen des brevets

(11) **EP 4 406 556 A1**

(12) **EUROPEAN PATENT APPLICATION**
published in accordance with Art. 153(4) EPC

(43) Date of publication:
**31.07.2024 Bulletin 2024/31**

(21) Application number: **22872982.8**

(22) Date of filing: **22.09.2022**

(51) International Patent Classification (IPC):
*A61K 41/00* $^{(2020.01)}$ *A61K 31/409* $^{(2006.01)}$
*A61K 38/02* $^{(2006.01)}$ *A61K 39/395* $^{(2006.01)}$
*A61K 45/00* $^{(2006.01)}$ *A61K 47/64* $^{(2017.01)}$
*A61K 47/68* $^{(2017.01)}$ *A61P 35/00* $^{(2006.01)}$

(52) Cooperative Patent Classification (CPC):
**A61K 31/409; A61K 38/02; A61K 39/395;
A61K 41/00; A61K 45/00; A61K 47/64;
A61K 47/68; A61P 35/00**

(86) International application number:
**PCT/JP2022/035376**

(87) International publication number:
**WO 2023/048231 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.09.2021 JP 2021155009**

(71) Applicants:
• **PhotoQ3 Inc.**
 **Tokyo 102-0084 (JP)**
• **Nippon Medical School Foundation**
 **Tokyo 113-8602 (JP)**

(72) Inventors:
• **HAMAKUBO, Takao**
 **Tokyo 102-0084 (JP)**
• **TODA, Naoko**
 **Tokyo 102-0084 (JP)**
• **SUDO, Yukio**
 **(Deceased) (JP)**
• **USUDA, Jitsuo**
 **Tokyo 113-8602 (JP)**
• **SONOKAWA, Takumi**
 **Tokyo 113-8602 (JP)**

(74) Representative: **Hoffmann Eitle
Patent- und Rechtsanwälte PartmbB
Arabellastraße 30
81925 München (DE)**

(54) **MEDICINE FOR KILLING TUMOR CELLS**

(57)   It is an object of the present invention to provide a medicament for killing tumor cells, having few side effects. According to the present invention, provided is a medicament for killing tumor cells, comprising: a conjugate of a substance that binds to a target substance on the surface of tumor cells and a cytotoxin; and Talaporfin Sodium, Porfimer Sodium or Verteporfin, wherein Talaporfin Sodium, Porfimer Sodium or Verteporfin is administered 1 to 4 days after administration of the conjugate of a substance that binds to a target substance on the surface of tumor cells and a cytotoxin, and a wavelength effective for activating Talaporfin Sodium, Porfimer Sodium or Verteporfin is irradiated 1 to 4 hours after the administration of Talaporfin Sodium, Porfimer Sodium or Verteporfin.

[Fig. 1]

**Description**

Technical Field

The present invention relates to a medicament for killing tumor cells, comprising a conjugate of a substance that binds to a target substance on the surface of tumor cells and a cytotoxin, and Talaporfin Sodium, Porfimer Sodium or Verteporfin.

Background Art

In Japan, approximately 370,000 people die of cancer each year, and cancer has continuously been a leading cause of death since 1981. To date, a large number of therapeutic methods for cancer have been developed inside and outside of the country. However, a specific remedy for cancer has not yet been developed.

What is called, small molecule anticancer agents (cancer chemotherapy) have been developed. However, the medicinal effects of these anticancer agents are not strong enough, and further, severe side effects cause trouble to patients. Thus, it cannot be said that these anticancer agents are desirable medicaments. Since antibody drugs are characterized in that the antibody drugs have strong specificity to cancer, strong side effects found in the small molecule anticancer agents can be alleviated, and thus, such antibody drugs can be broadly used. However, there are yet only a small number of antibody drugs effective for solid cancer. In order to reinforce medicinal effects, ADC (antibody-drug conjugate) has been developed, but such ADC has not yet been satisfactory, including its toxicity.

Moreover, an immunotherapeutic antibody (checkpoint inhibitor) used as a novel antibody drug exhibits strong medicinal effects against a wide range of cancer species based on its novel mechanism. However, it has been found that the number of patients who receive the effects of such an immunotherapeutic antibody is not necessarily large, and that, in some cases, the patients have severe side effects to such an extent that they lead to death.

On the other hand, on the basis of an idea that the therapeutic site is limited and side effects are thereby reduced, PDT (Photo Dynamic Therapy) has been developed. PDT is a therapeutic method by which a light having a certain wavelength that activates photosensitizing dyes gathering in a tumor site is applied to an affected area to treat it. PDT shows certain effects against lung cancer, etc., but it cannot be said that satisfactory medicinal effects are obtained from this therapeutic method.

Summary of Invention

Objects to be Solved by the Invention

Under such circumstances, the present inventors have felt a need to develop a pharmaceutical product having few side effects and strong medicinal effects, and thereby achieving the present development goals. It is an object of the present invention to provide a medicament for killing tumor cells, having few side effects, and a method of administration thereof.

Means for Solving the Objects

PDT is a method for destroying tumor cells by accumulating sensitizing dyes to tumor tissues and then applying a light to the tumor cells. As such sensitizing dyes used in PDT, sensitizing dyes having high water-solubility and a high property of accumulating in tumor, Talaporfin Sodium, Porfimer Sodium or Verteporfin, have been known, and all of these sensitizing dyes have already been used in the treatment of lung cancer and the like. PDT is a low-invasive therapeutic method, but this method is disadvantageous in that the medicinal effects thereof are not necessarily satisfactory.

PCI (Photochemical Internalization) is a method for destroying an endosomal membrane by accumulating photosensitizers to an endosomal membrane and then applying a light to the endosomal membrane. It is considered that, according to PCI, anticancer agents or immunotoxins enclosed in the endosomal membrane are released into the cytoplasm, so that tumor cells are destroyed. For the purpose of accumulating photosensitizers to the endosomal membrane, the dyes used in PCI are different from those used in PDT, and amphipathic photosensitizers such as sulfonated tetraphenylchlorin (TPCS2a) or aluminum phthalocyanine (AlPcS2a) are used.

However, such amphipathic sulfonated tetraphenylchlorin (TPCS2a) or aluminum phthalocyanine (AlPcS2a) is disadvantageous in that, for example, (1) neurotoxicity is concerned, in that (2) the photosensitizers accumulate in cell membranes other than the endosomal membrane and cause cytotoxicity, and in that (3) the photosensitizers have a low property of accumulating in tumor and cause non-specific side effects.

The present inventors have conducted intensive studies directed towards solving the previous problems. As a result, the present inventors have found that Talaporfin Sodium, Porfimer Sodium or Verteporfin which are highly water-soluble, has such unpredictable effects that it increases the permeability of the endosomal membrane even at a low concentration. Moreover, the present inventors have also found that, by combining a conjugate of a substance that binds to a target substance on the surface of tumor cells and a cytotoxin, with Talaporfin Sodium, Porfimer Sodium or Verteporfin, cytotoxicity and tumor specificity can be significantly reinforced, thereby completing the present invention.

In the above method, when the photosensitizer and the conjugate of a substance that binds to a target substance on the surface of tumor cells and a cytotoxin are simultaneously collect in endosomes at the time of light irradiation, the effect can be achieved. However, although a photosensitizing dye which has a high metabolic rate, is effective in reducing side effects, it is not easy to simultaneously accumulate it with conjugate of a substance that binds to a target substance on the surface of tumor cells and a cytotoxin such as immunotoxin. Therefore, we thought it necessary to consider a different administration method. As a result of our research for such conditions, we have found a method wherein a substance that binds to a target substance on the surface of tumor cells, such as immunotoxin, is administered, and then left for several days to accumulate it in the tumor, and then a photosensitizer is administered and light irradiation is performed several hours later, thereby completing the present invention.

According to the present invention, the following inventions are provided.

<1> A medicament for killing tumor cells, comprising:

a conjugate of a substance that binds to a target substance on the surface of tumor cells and a cytotoxin; and Talaporfin Sodium, Porfimer Sodium or Verteporfin,

wherein

Talaporfin Sodium, Porfimer Sodium or Verteporfin is administered 1 to 4 days after administration of the conjugate of a substance that binds to a target substance on the surface of tumor cells and a cytotoxin, and a wavelength effective for activating Talaporfin Sodium, Porfimer Sodium or Verteporfin is irradiated 1 to 4 hours after the administration of Talaporfin Sodium, Porfimer Sodium or Verteporfin.

<2> The medicament according to <1>, wherein the substance that binds to a target substance on the surface of tumor cells is an antibody, an antibody fragment, a ligand, or a peptide.

<3> The medicament according to <1> or <2>, wherein the cytotoxin is saporin, gelonin, or Pseudomonas exotoxin.

<4> The medicament according to any one of <1> to <3>, wherein the tumor cells are cells that express Epidermal Growth Factor Receptor (EGFR, ERBB1, ERBB2, ERBB3, or ERBB4), Mesothelin, Ephrin type-A receptor 2 (EphA2), Glypican 3 (GPC3), Cadherin 17 (CDH17), or Roundabout homolog 1 (Robo1) on the cell surface thereof.

<5> The medicament according to any one of <1> to <4>, wherein the tumor cells are cancer cells of any one of head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.

<6> The medicament according to any one of <1> to <5>, wherein the wavelength effective for activating Talaporfin Sodium, Porfimer Sodium or Verteporfin is 600 to 800 nm.

< A > A method for killing tumor cells, comprising:

(1) a step of allowing a conjugate of a substance that binds to a target substance on the surface of tumor cells and a cytotoxin to come into contact with tumor cells;
(2) a step of allowing Talaporfin Sodium, Porfimer Sodium or Verteporfin to come into contact with the tumor cells 1 to 4 days after the step (1) was carried out; and
(3) a step of irradiating the tumor cells with a wavelength effective for activating Talaporfin Sodium, Porfimer Sodium or Verteporfin 1 to 4 hour after the step (1) was carried out, so as to kill the cells, .

Advantageous Effects of Invention

According to the present invention, a medicament for killing tumor cells, having few side effects, can be provided.

Brief Description of Drawings

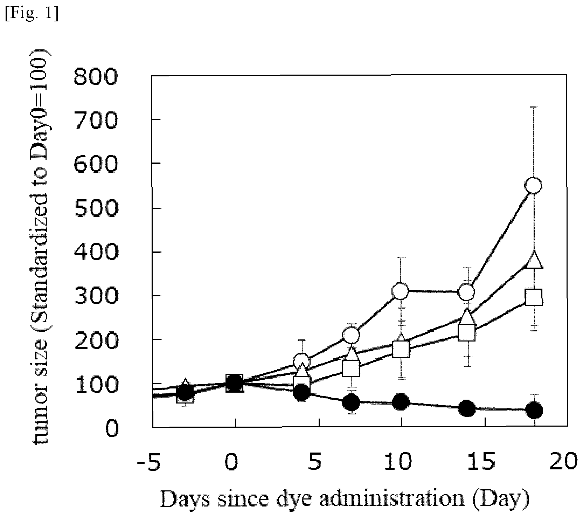

[Fig. 1] Fig. 1 shows the results of tumor regression effect in carcinoma-bearing mice.

Embodiment of Carrying out the Invention

Hereinafter, the embodiments of the present invention will be described in detail.

< Summary of the Invention >

The present inventors have studied a method for treating a tumor, which has strong medicinal effects and few side effects, and as a result, they have conceived that the techniques PDT and PCI that topically enhance medicinal effects as a result of light irradiation have potential. However, PDT has been disadvantageous in terms of weak medicinal effects, whereas PCI has not been satisfactory in terms of both medicinal effects and toxicity.

Hence, the present inventors have conducted intensive studies, and as a result, they have found for the first time that a water-soluble sensitizing dye, namely, Talaporfin Sodium, Porfimer Sodium or Verteporfin improves the permeability of the endosome by light irradiation. By combining this "dye," "a conjugate of a substance that binds to a target substance on the surface of tumor cells and a cytotoxin," and "light irradiation to a tumor," the present inventors have discovered a therapeutic method having strong medicinal effects and few side effects, thereby completing the present invention.

Specifically, in the present invention, a conjugate of a substance that binds to a target substance on the surface of tumor cells and a cytotoxin binds to a tumor, and it is then enclosed in the endosome. It is conceived that a light is then applied to Talaporfin Sodium, Porfimer Sodium or Verteporfin, which has been added separately (or simultaneously), so that an immunotoxin (or a decomposed product thereof) in the endosome is released into the cytoplasm, and thereby, the tumor cells can be killed.

Talaporfin Sodium, Porfimer Sodium and Verteporfin have the advantage of being water soluble and having fewer side effect concerns in localizing to membranes such as amphiphilic sulfonated tetraphenylclorine (TPCS2a) and aluminum phthalocyanine (AlPcS2a). Furthermore, the absorption wavelength is 664 nm, which does not overlap with the absorption wavelength of hemoglobin, allowing the light to reach a greater depth.

The wavelength for activating Talaporfin Sodium, Porfimer Sodium or Verteporfin is preferably 600 to 800 nm, more referably 600 to 750 nm, further preferably 600 to 700 nm, and particularly preferably 650 to 680 nm.

<Method for Killing Cells >

In the present invention, (1) a conjugate of a substance that binds to a target substance on the surface of tumor cells and a cytotoxin is administered, and then, (2) Talaporfin Sodium, Porfimer Sodium or Verteporfin is administered, and then, (3) a wavelength effective for activating Talaporfin Sodium, Porfimer Sodium or Verteporfin is irradiated.

The interval between administration of the substance that binds to the target substance on the surface of tumor cells and administration of Talaporfin Sodium, Porfimer Sodium or Verteporfin is 1 to 4 days, preferably 1 to 3 days, for example 2 days.

The interval between administration of Talaporfin Sodium, Porfimer Sodium or Verteporfin and light irradiation is 1 to 4 hours, preferably 1 to 3 hours, for example 2 hours.

< Photosensitizing Dye >

In the present invention, Talaporfin Sodium, Porfimer Sodium or Verteporfin is used as a sensitizer.

Talaporfin Sodium is a sensitizing dye used in PDT, which is also referred to as Laserphyrin, NPe6, or mono-aspartyl chlorin e6.

Furthermore, as another embodiment, Porfimer Sodium can also be used as a sensitizing dye. Porfimer Sodium is a PDT dye that is also referred to as Photofrin.

Moreover, as another embodiment, Verteporfin can also be used as a sensitizing dye. Verteporfin is a PDT dye that is also referred to as Visudyne.

< Substance that Binds to Target Substance on Surface of Tumor Cells >

Examples of the substance that binds to a target substance on the surface of tumor cells may include, but are not particularly limited to, an antibody, an antibody fragment, a ligand, and a peptide.

When an antibody is used as such a substance that binds to a target substance on the surface of tumor cells, it is possible to use an antibody that specifically binds to a target substance on the surface of tumor cells (for example, a protein, such as Epidermal Growth Factor Receptor (EGFR, ERBB1, ERBB2, ERBB3, or ERBB4), Mesothelin, Ephrin type-A receptor 2 (EphA2), Glypican 3 (GPC3), Cadhelin 17 (CDH17), Cadherin 3 (CDH3), or Roundabout homolog 1 (Robot)).

The type of the antibody used in the present invention is not particularly limited, and examples of the present antibody may include a mouse antibody, a human antibody, a rat antibody, a rabbit antibody, a sheep antibody, a camel antibody, an avian antibody, and a genetically modified antibody that is artificially modified for the purpose of reducing xenoantigenicity against a human, such as a chimeric antibody or a humanized antibody. Such a genetically modified antibody can be produced by applying a known method. The chimeric antibody is an antibody consisting of the heavy chain and light chain variable regions of a mammalian antibody other than a human antibody, such as a mouse antibody, and the heavy chain and light chain constant regions of a human antibody. The chimeric antibody can be obtained by ligating DNA encoding the variable region of a mouse antibody to DNA encoding the constant region of a human antibody, then incorporating the ligate into an expression vector, and then introducing the expression vector into a host, so that the host is allowed to generate the antibody. The humanized antibody is obtained by transplanting the complementarity determining region (CDR) of a mammalian antibody other than a human antibody, such as a mouse antibody, into the complementarity determining region of a human antibody. A common gene recombination method therefor has been known. Specifically, a DNA sequence designed to ligate the CDR of a mouse antibody to the framework region (FR) of a human antibody is synthesized from several oligonucleotides that have been produced such that they have an overlapping portion at the terminal portions thereof according to a PCR method. The obtained DNA is ligated to DNA encoding the constant region of a human antibody, and the ligate is then incorporated into an expression vector, which is then introduced into a host, so that the host is allowed to generate the antibody (EP 239400, International Publication WO96/02576, etc.).

In addition, a method of obtaining a human antibody has also been known. For example, human lymphocytes are sensitized with a desired antigen or a cell expressing the desired antigen *in vitro,* and then fusing the sensitized lymphocytes with human myeloma cells, such as, for example, U266, so as to obtain a desired human antibody having a binding activity to an antigen (JP Paten Publication (Kokoku) No. 1-59878 B (1989)). Otherwise, a transgenic antibody having all repertoires of human antibody genes is immunized with a desired antigen to obtain a desired human antibody (see WO93/12227, WO92/03918, WO94/02602, WO94/25585, WO96/34096, and WO96/33735). Further, a technique of obtaining a human antibody by panning using a human antibody library has also been known. For example, a human antibody variable region is allowed to express as a single chain antibody (scFv) on the surface of a phage according to a phage display method, and a phage binding to an antigen can be then selected. By analyzing the selected phage gene, a DNA sequence encoding the variable region of a human antibody binding to the antigen can be determined. If the DNA sequence of scFv binding to an antigen is clarified, a suitable expression vector comprising the sequence can be produced, so that a human antibody can be obtained. These methods have already been publicly known, and please refer to WO92/01047, WO92/20791, WO93/06213, WO93/11236, WO93/19172, WO95/01438, and WO95/15388.

The antibody that binds to tumor cells is preferably a humanized or a human antibody, but is not limited thereto.

Moreover, these antibodies may also be low molecular weight antibodies such as antibody fragments, or modified forms of the antibodies, unless they lose the property of recognizing the entire or a part of a protein encoded by an antigen gene present on the surface of tumor cells. The antibody fragment is a part of an antibody that retains a binding ability to ROBO1. Specific examples of the antibody fragment may include Fab, Fab', F(ab')2, Fv, Diabody, and a single chain variable fragment (scFv). In order to obtain such an antibody fragment, a gene encoding such an antibody fragment is constructed, the gene is then introduced into an expression vector, and it may be then expressed in suitable host cells. As a modified form of an antibody, an antibody binding to various types of molecules such as polyethylene glycol (PEG) can also be used.

DNA encoding a monoclonal antibody can be easily isolated and sequenced according to a commonly used method (for example, by using an oligonucleotide probe capable of specifically binding to a gene encoding the heavy chain and light chain of the monoclonal antibody). Hybridoma cells may be preferable starting materials for such DNA. Once such DNA is isolated, it is inserted into an expression vector, and the expression vector is then used to transform host cells such as *E. coli* cells, COS cells, CHO cells, or myeloma cells that do not generate immunoglobulin before they are transformed. Then, a monoclonal antibody can be generated from the transformed host cells.

As a substance that binds to a target substance on the surface of tumor cells, a ligand can be used. When the target substance on the surface of tumor cells is, for example, a receptor such as Epidermal Growth Factor Receptor (EGFR, ERBB1, ERBB2, ERBB3, or ERBB4), Mesothelin, or Ephrin type-A receptor 2 (EphA2), a ligand against each of the above-described receptors can be used.

As such a substance that binds to a target substance on the surface of tumor cells, a peptide can also be used. A peptide that binds to a target substance on the surface of tumor cells can be designed and produced by those skilled in the art.

< Cytotoxin >

The cytotoxin is preferably a protein having cytotoxicity, but is not limited thereto. The cytotoxin may also be a compound having a synthetic or natural anticancer action, such as bleomycin, or a compound used in ADC.

Preferred examples of such a protein having cytotoxicity may include saporin, gelonin, Pseudomonas exotoxin, ricin A chain, deglycosylated ricin A chain, a ribosome inactivating protein, alphasarcine, aspergillin, restrictocin, ribonuclease, epipodophyllotoxin, diphtheria toxin, Shigatoxin, and a mutant or a genetically modified body thereof.

< Conjugate of Substance that Binds to Target Substance on Surface of Tumor Cells and Cytotoxin >

A substance that binds to a target substance on the surface of tumor cells, and a cytotoxin, must bind to each other, directly or indirectly.

When an antibody or a fragment thereof is used as such a substance that binds to a target substance on the surface of tumor cells, as a method of directly chemically binding the antibody or the fragment thereof to a cytotoxin, a binding method used for known ADC (Antibody Drug Conjugate) can be used. Otherwise, when the cytotoxin is a protein, a bifunctional crosslinking agent can also be used.

Alternatively, when the cytotoxin is a protein, a toxin is fused with an antibody or a fragment thereof by genetic recombination to form a protein, so that an immunotoxin can be produced.

Moreover, as another method, a method of indirectly binding an antibody or a fragment thereof to a cytotoxin by using a second binding pair can also be used. Examples of the second binding pair that can be utilized herein may include avidin-biotin and an antibody-hapten.

Further, in the present invention, it is also possible to use a conjugate of a peptide or a ligand that binds to a target substance on the surface of tumor cells and a toxin, instead of using an immunotoxin in which an antibody and a toxin bind to each other.

< Administration Methods and Applied Doses >

The method for administering the medicament of the present invention to a subject having a tumor (for example, a cancer, etc.) is not particularly limited.

The conjugate of a substance that binds to a target substance on the surface of tumor cells and a cytotoxin can be administered to the subject, for example, via intravenous administration, arterial administration, intramuscular administration, subcutaneous administration, intradermal administration, intraperitoneal administration, or oral administration. Alternatively, an administration method involving administration of the conjugate to tumor tissues and the periphery thereof via local injection, application, spraying or the like can also be applied.

Talaporfin Sodium, Porfimer Sodium or Verteporfin can be administered to a subject, for example, via intravenous administration, arterial administration, intramuscular administration, subcutaneous administration, intradermal administration, intraperitoneal administration, or oral administration. Alternatively, an administration method involving administration to tumor tissues and the periphery thereof via local injection, application, spraying or the like can also be applied.

The applied dose of the conjugate of a substance that binds to a target substance on the surface of tumor cells and a cytotoxin is not particularly limited. The conjugate can be administered to a subject at a dose of, for example, 1 $\mu$g/kg of body weight to 100 mg/kg of body weight, and preferably, at a dose of 10 $\mu$g/kg of body weight to 10 mg/kg of body weight.

The applied dose of Talaporfin Sodium, Porfimer Sodium or Verteporfin is not particularly limited. It can be administered to a subject at a dose of, for example, 1 $\mu$g/kg of body weight to 100 mg/kg of body weight, and preferably, at a dose of 10 $\mu$g/kg of body weight to 10 mg/kg of body weight.

The number of doses is not particularly limited, and administration can be carried out once to several times (from once to 20 times, and preferably from once to 10 times). Administration can be carried out, for example, every 2 to 4 weeks, or every 1 to 2 months. In addition, the number of light irradiation operations is not particularly limited, either. The light irradiation can be carried out once to several times.

< Cells and/or Diseases as Targets >

The tumor as a target of the administration of the medicament of the present invention is a tumor that expresses Epidermal Growth Factor Receptor (EGFR, ERBB 1, ERBB2, ERBB3, or ERBB4), Mesothelin, Ephrin type-A receptor 2 (EphA2), Glypican 3 (GPC3), Cadhelin 17 (CDH17), Cadhelin 3 (CDH3), Roundabout homolog 1 (Robo1) or the like on the surface thereof.

Specific examples of the tumor as a target of the administration of the medicament of the present invention may include cancers, such as head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.

Moreover, the present invention can be used in the treatment of animals other than humans, such as dogs, cats or horses, as well as the treatment of the diseases of humans.

Examples

Example : Confirmation of tumor regression effect in tumor-bearing mice by iTAP treatment using IT-Cetuximab and Laserphyrin

< Acquisition of Materials >

As an EGFR-expressing cell line, A549 (human lung cancer cell) was acquired from KAC Company (Kyoto, Japan). As an anti-EGFR antibody, Cetuximab was acquired from Selleck Biotech, Ltd. (Tokyo, Japan). Laserpyhrin was acquired from Meiji Seika Pharma Co., Ltd. (Tokyo, Japan).

< Adjustment of Immunotoxin >

Cetuximab dissolved in PBS(-) was mixed with EZ-LINK sulfo-NHS-LC-biotinylation reagent (Thermo Fisher Scientific, Commonwealth of Massachusetts) dissolved in ultrapure water at a molar ratio of 1 : 40, and the obtained mixture was then reacted. Thereafter, the reaction mixture was purified using PD SpinTrap G-25 (GE Healthcare Life Sciences, England). The biotinylated Cetuximab was equivalently mixed with streptavidin-saporin (Biotin-Z Internalization Kit [KIT-27-Z], Advanced Targeting Systems, California), and the obtained mixture was reacted at room temperature for 30 minutes, so as to obtain saporin-conjugated Cetuximab (IT-Cetuximab).

< Cell Culture >

Using a medium prepared by adding 10% fetal bovine serum to a high-glucose Dulbecco's Modified Eagle's Medium (DMEM), A549 was cultured under conditions of 37°C and 5% $CO_2$ concentration.

<Preparation of A549 cell line xenograft mouse>

Separately cultured A549 cells were adjusted to $1 \times 10^7$ cells/100 μL, and the cells were subcutaneously administered (SC) to the right thigh of 7-week-old male BALB/c Slc-nu/nu mice, and xenograft mice (tumor-bearing mice) were produced. Tumor size was calculated using the following formula.

$$\text{Tumor volume (mm}^3) = \text{major axis (mm)} \times \text{minor axis (mm)} \times \text{minor axis (mm)} \times 0.5$$

<Study of tumor regression effect in tumor-bearing mice by iTAP treatment using IT-Cetuximab and Laserphyrin>

1. Administration and irradiation

When the tumor size of the mice reached an average size of approximately 150 mm³, the mice were randomly divided into the following groups (n=3).
Condition (1) Control group: No administration
Condition (2) Laserphyrin (5mg/kg) administration and 664 nm laser light (30J/cm²) irradiation group
Condition (3) IT-Cetuximab (3mg/kg) administration group
Condition (4) Laserphyrin (5mg/kg), IT-Cetuximab (3mg/kg) administration and 664 nm laser light (30J/cm²) irradiation group

IT-Cetuximab was administered intraperitoneally to mice, and 2 days later, Laserphyrin was administered through the tail vein. Furthermore, 2 hours after the administration of Laserphyrin, the tumor site was locally irradiated with a 664 nm laser light.

2. observation

Tumor size was measured every 3 to 5 days after administration.

<Results>

The measurement results of tumor size after administration are shown in Figure 1.

In (4) the IT-Cetuximab + Laserphyrin combination group, significant suppression of tumor growth was observed compared to (1) the control group, (2) the Laserphyrin alone group, or (3) the IT-Cetuximab alone group.

In addition, tumor growth was confirmed macroscopically in (1) the control group, (2) the Laserphyrin alone group, or (3) the IT-Cetuximab alone group, whereas (4) IT-Cetuximab + Laserphyrin combination group, it was confirmed that a eschar formed at the irradiated site several days after laser irradiation, and that the tumor has regressed as the eschar fell off.

These results revealed that the tumor suppressing effect of this method was remarkable.

**Claims**

1. A medicament for killing tumor cells, comprising:

   a conjugate of a substance that binds to a target substance on the surface of tumor cells and a cytotoxin; and
   Talaporfin Sodium, Porfimer Sodium or Verteporfin,

   wherein

   Talaporfin Sodium, Porfimer Sodium or Verteporfin is administered 1 to 4 days after administration of the conjugate of a substance that binds to a target substance on the surface of tumor cells and a cytotoxin, and a wavelength effective for activating Talaporfin Sodium, Porfimer Sodium or Verteporfin is irradiated 1 to 4 hours after the administration of Talaporfin Sodium, Porfimer Sodium or Verteporfin.

2. The medicament according to claim 1, wherein the substance that binds to a target substance on the surface of tumor cells is an antibody, an antibody fragment, a ligand, or a peptide.

3. The medicament according to claim 1 or 2, wherein the cytotoxin is saporin, gelonin, or Pseudomonas exotoxin.

4. The medicament according to any one of claims 1 to 3, wherein the tumor cells are cells that express Epidermal Growth Factor Receptor (EGFR, ERBB 1, ERBB2, ERBB3, or ERBB4), Mesothelin, Ephrin type-A receptor 2 (EphA2), Glypican 3 (GPC3), Cadherin 17 (CDH17), or Roundabout homolog 1 (Robo1) on the cell surface thereof.

5. The medicament according to any one of claims 1 to 4, wherein the tumor cells are cancer cells of any one of head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.

6. The medicament according to any one of claims 1 to 5, wherein the wavelength effective for activating Talaporfin Sodium, Porfimer Sodium or Verteporfin is 600 to 800 nm.

[Fig. 1]

| INTERNATIONAL SEARCH REPORT | International application No. |
|---|---|
| | **PCT/JP2022/035376** |

**A. CLASSIFICATION OF SUBJECT MATTER**

*A61K 41/00*(2020.01)i; *A61K 31/409*(2006.01)i; *A61K 38/02*(2006.01)i; *A61K 39/395*(2006.01)i; *A61K 45/00*(2006.01)i; *A61K 47/64*(2017.01)i; *A61K 47/68*(2017.01)i; *A61P 35/00*(2006.01)i
FI: A61K41/00; A61K31/409; A61K38/02; A61K39/395 C; A61K39/395 E; A61K39/395 L; A61K39/395 T; A61K39/395 Y; A61K45/00; A61K47/64; A61K47/68; A61P35/00

According to International Patent Classification (IPC) or to both national classification and IPC

**B. FIELDS SEARCHED**

Minimum documentation searched (classification system followed by classification symbols)

A61K41/00; A61K31/409; A61K38/02; A61K39/395; A61K45/00; A61K47/64; A61K47/68; A61P35/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | 浜窪隆雄, 光感受性色素とイムノトキシンを用いた新規がん治療法（iTAP）法の開発, 工業材料, 01 August 2021, vol. 69, no. 8, pp. 50-54, ISSN 0452-2834<br>pp. 50-54, (HAMAKUBO, Takao. Engineering materials.), non-official translation (Development of a novel cancer treatment (iTAP) method using photosensitive dye and immunotoxin) | 1-6 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 November 2022** | **29 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 239400 A
- WO 9602576 A
- JP 1059878 B
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A